Europäisches Patentamt

European Patent Office

Office européen des brevets

(19)

(11) Veröffentlichungsnummer: **0 008 052**
**B1**

(12) **EUROPÄISCHE PATENTSCHRIFT**

(45) Veröffentlichungstag der Patentschrift:
**13.02.85**

(21) Anmeldenummer: **79102700.6**

(22) Anmeldetag: **30.07.79**

(51) Int. Cl.⁴: **A 61 K 33/42** // (A61K33/42, 33/14, 33/08, 31/725, 31/045),(A61K33/42, 33/14, 31/19)

(54) **Heilmittel mit entzündungshemmender und blutstillender Wirkung.**

(30) Priorität: **04.08.78 DE 2834320**

(43) Veröffentlichungstag der Anmeldung:
**20.02.80 Patentblatt 80/4**

(45) Bekanntmachung des Hinweises auf die Patenterteilung:
**13.02.85 Patentblatt 85/7**

(84) Benannte Vertragsstaaten:
**AT CH FR GB IT**

(56) Entgegenhaltungen:
**DE - A - 2 351 512**

**Die Akte enthält technische Angaben, die nach dem Eingang der Anmeldung eingereicht wurden und die nicht in dieser Patentschrift enthalten sind.**

(73) Patentinhaber: **Tomic, Dobrivoje, Dr., Fasangartenstrasse 159, D-8000 München 90 (DE)**

(72) Erfinder: **Tomic, Dobrivoje, Dr., Fasangartenstrasse 159, D-8000 München 90 (DE)**

## Beschreibung

Die Erfindung betrifft ein Mittel mit heilender, entzündungshemmender und blutstillender Wirkung, das zur Behandlung von Wunden und Erkrankungen der weichen und harten Gewebe anwendbar ist und das die in den Ansprüchen angegebene Zusammensetzung aufweist.

Die Grundlage für bekannte, zur Entzündungshemmung eingesetzte Antiphlogistika bilden in der Regel Corticosteroide, Pyrazolidine und Bakteriostatika vom Typ der Antibiotika und Sulfonamide. Die bekannten Blutstillungsmittel basieren vorwiegend auf Vasokonstringentien vom Typ des Adrenalins und Noradrenalins, auf gerinnungshemmenden Stoffen vom Typ des Thrombins und Fibrinogens und auf saugfähigen Substanzen vom Typ der Oxycellulose und Gelatineschwämme.

Diese bekannten Mittel mit heilender bzw. entzündungshemmender und blutstillender Wirkung sind jedoch mit zahlreichen Nachteilen verbunden. Die Heilwirkung ist oft von der Haftfähigkeit dieser Mittel abhängig, insbesondere z. B. an Haut und Schleimhaut. Oft haben die bekannten Mittel den Nachteil, daß, wenn überhaupt, nur bei langfristiger Behandlung oftmals Gewebeschädigungen und Allergien die Folge sind. Bei Einsatz von Bakteriostatika wird oft die Entstehung von resistenten Erregerstämmen begünstigt. In der Regel sind alle Mittel die keine Bakteriostatika enthalten nur blutstillend oder entzündungshemmend (DE-A-2 351 512).

Aufgabe der Erfindung ist es, Mittel zur Behandlung von weichem und hartem Gewebe mit heilender, entzündungshemmender und blutstillender Wirkung bereitzustellen, die sich durch rasche Wirkung, ausgezeichnete Verträglichkeit und gute Haftung auszeichnen und auch bei Daueranwendung völlig unbedenklich sind, so daß sie sich ganz besonders zur Wundheilung und Blutstillung eignen.

Der Erfindung liegt eine spezielle Zusammensetzung zugrunde. Sie weist einen vergleichweise niedrigen pH-Wert und einen vergleichweise hohen osmotischen Druck auf. Mit der erfindungsgemäßen Zusammensetzung wird innerhalb kürzester Zeit, meistens sogar bereits innerhalb weniger Minuten eine Blutstillung und Entzündungshemmung erreicht. Der pH-Wert wird durch Beifügen von adstringierenden Mitteln oder Säure eingestellt.

Das erfindungsgemäße Mittel hat die Konsistenz ähnlich einer Emulsion, Paste, Creme, Salbe oder als Lösung.

Die Herstellung des erfindungsgemäßen Mittels erfolgt durch Einverleiben und Vermischen der einzelnen Komponenten in bekannter, auf pharmazeutischem Gebiet üblicher Weise. Zur Herstellung des Mittels wird von den angegebenen Komponenten ausgegangen, welche ggf. in vorgemischter Form, in der dem Fachmann geläufigen Weise einverleibt werden.

Das erfindungsgemäße Mittel eignet sich hervorragennd zur Behandlung von Entzündungen, Blutungen, Verbrennungen, Wunden aller Art und Erkrankungen der Haut und Schleimhaut.

Die folgenden Beispiele sollen die Erfindung näher erläutern.

Beispiel 1

| Komponente | Gehalt | ungefährer Gehalt in % |
|---|---|---|
| NaCl | 6,7 g | 0,7 |
| KCl | 0,17 g | 0,02 |
| $Na_2HPO_4 \cdot 12 H_2O$ | 6,12 g | 0,6 |
| $MgCl_2 \cdot 6 H_2O$ | 0,17 g | 0,02 |
| $Ca_3(PO_4)_2$ | 60,0 g | 6 |
| Pektin | 60,0 g | 6 |
| Glycerin | 120,0 g | 12 |
| MgO | 2,0 g | 0,2 |
| $H_2O$ | ad 1 l | ad 100 |

2

Beispiel 2

| Komponente | Gehalt | ungefährer Gehalt in % |
|---|---|---|
| Aluminiumacetattartrat | 50,0 g | 5 |
| NaCl | 7,0 g | 0,7 |
| KCl | 0,17 g | 0,02 |
| $Na_2HPO_4 \cdot 12 H_2O$ | 6,0 g | 0,6 |
| $MgCl_2 \cdot 6 H_2O$ | 0,17 g | 0,02 |
| $H_2O$ | ad 1 l | ad 100 |

Zu den Beispielen 1 und 2 wird je nach Anwendungsbereich wie z. B. bei Parodontopathien ein Geschmackmittel (z. B. Pfefferminzöl) und/oder ein Adstringent (z. B. Tannin, Alu.act.) bzw. Säure (z. B. Phosphorsäure, Citronensäure) beigemischt.

**Patentansprüche**

1. Heilmittel zur Behandlung von Wunden, Entzündungen und Erkrankungen der weichen und harten Gewebe und gegen Blutungen, dadurch gekennzeichnet, daß

| | |
|---|---|
| 0,67 — 0,7 Gew.-% | NaCl |
| 0,017 — 0,02 Gew.-% | KCl |
| 0,6 — 0,612 Gew.-% | $Na_2HPO_4 \cdot 12 H_2O$ |
| 0,017 — 0,02 Gew.-% | $MgCl_2 \cdot 6 H_2O$ |
| 6 Gew.-% | $Ca_3(PO_4)_2$ |
| 6 Gew.-% | Pektin |
| 12 Gew.-% | Glycerin |
| 0,2 Gew.-% | MgO |

und Wasser ad 1000 Milliliter Zubereitung enthalten sind, wobei neben diesen Komponenten je nach Anwendungsbereich gegebenenfalls noch ein Geschmacksmittel und/oder ein adstringierendes Mittel bzw. eine Säure enthalten sein können.

2. Heilmittel zur Behandlung von Wunden, Entzündungen und Erkrankungen der weichen und harten Gewebe in Form einer Lösung, dadurch gekennzeichnet, daß

| | |
|---|---|
| 5 Gew.-% | Aluminiumacetattartrat |
| 0,7 Gew.-% | NaCl |
| 0,017—0,02 Gew.-% | KCl |
| 0,6 Gew.-% | $Na_2PO_4 \cdot 12 H_2O$ |
| 0,017—0,02 Gew.-% | $MgCl_2 \cdot 6 H_2O$ |

und Wasser ad 1000 Milliliter Zubereitung enthalten sind, wobei neben diesen Komponenten je nach Anwendungsbereich gegebenenfalls noch ein Geschmacksmittel und/oder ein adstringierendes Mittel bzw. eine Säure enthalten sein können.

**Claims**

1. Remedy for the treatment of wounds, inflammations, and illnesses of the soft and hard tissues and against hemorrhages, marked by the following ingredient in the following approximate percentage amounts for a 1000 ml preparation therof,

| | |
|---|---|
| 0,67 — 0,7 g — % | NaCl |
| 0,017 — 0,02 g — % | KCl |
| 0,6 — 0,612 g — % | $Na_2HPO_4 \cdot 12 H_2O$ |
| 0,017 — 0,02 g — % | $MgCl_2 \cdot 6 H_2O$ |
| 6 g — % | $Ca_3(PO_4)_2$ |
| 6 g — % | Pektin |
| 12 g — % | Glycerin |
| 0,2 g — % | MgO |

H2O add 1000 ml, wherby in addition to these ingredients a flavor and/or an astringent or an acid may be added depending upon the range of application.

2. Remedy for the treatment of wounds, inflamations, and illnesses of the soft and hard tissues, having the consistency of a solution, marked by the following ingredients in the following approcimate amounts for a 1000 ml preparation thereof,

| | |
|---|---|
| 5 g — % | Aluminiumacetattartrat |
| 0,7 g — % | NaCl |
| 0,017 — 0,02 g — % | KCl |
| 0,6 g — % | $Na_2PO_4 \cdot 12\ H_2O$ |

H2O add 1000 ml, whereby a flavor and/or an astringent or acid may be added depending upon the range of application.

## Revendications

1. Médicament pour le traitement de plaies, d'inflammations et de maladies des tissus délicats et fermes et contre les hémorragies, ayant comme caractéristiques, que sont contenus

| | |
|---|---|
| de 0,67 à 0,7 de poids, — % | de NaCl |
| de 0,017 à 0,02 de poids, — % | de KCl |
| de 0,6 à 0,612 de poids, — % | de $Na_2HPC_4 \cdot 12\ H_2O$ |
| de 0,017 à 0,02 de poids, — % | de $MgCl_2 \cdot 6\ H_2O$ |
| 6 de poids, — % | de $Ca_3(PO_4)_2$ |
| 6 de poids, — % | de pectine |
| 12 de poids, — % | de glycérine |
| 0,2 de poids, — % | de MgO |

et de l'eau jusqu'à 1000 millilitres de préparation, alors que, selon la domaine d'application, le cas échéant, à part de ces composants, des goûts et/ou un astringent, respectivement un acide peuvent être contenus.

2. Médicament pour le traitement de plaies, d'inflammations et de maladies de tissus délicats et fermes sous forme de solution, ayant comme caractéristiques, que sont contenus

| | |
|---|---|
| 5 de poids, — % | de tartrate d'acétate d'aluminium |
| 0,7 de poids, — % | de NaCl |
| de 0,017 à 0,02 de poids, — % | de KCl |
| 0,6 de poids, — % | de $Na_2PO_4 \cdot 12\ H_2O$ |
| de 0,017 à 0,02 de poids, — % | de $MgCl_2 \cdot 6\ H_2O$ |

et de l'eau jusqu'à 1000 millilitres de préparation, alors que, selon le domaine d'application, le cas échéamt, à part de ces composants, des goûts et/ou un astringent, respectivement un acide peuvent être contenus.